## Europäisches Patentamt

## European Patent Office

## Office européen des brevets

(11) Publication number: **0 128 641**
A2

# EUROPEAN PATENT APPLICATION

(21) Application number: **84302399.5**

(22) Date of filing: **06.04.84**

(51) Int. Cl.³: **A 61 M 21/00, A 61 H 33/00**

(30) Priority: **06.04.83 GB 8309323**

(43) Date of publication of application: **19.12.84**
**Bulletin 84/51**

(84) Designated Contracting States: **AT CH DE FR LI NL**

(71) Applicant: **Allcock, John Anthony, 75 Windmill Road, Sunbury on Thames Middlesex (GB)**

(72) Inventor: **Allcock, John Anthony, 75 Windmill Road, Sunbury on Thames Middlesex (GB)**

(74) Representative: **Warren, Keith Stanley et al, BARON & WARREN 18 South End Kensington, London W8 5BU (GB)**

(54) **Isolation tank.**

(57) The present invention concerns an isolation tank for holding a heated saline solution is which users can float free from external stimuli. The tank is made from moulded plastics elements (1, 2, 4, 5 and 6) which together form a floatation chamber (8) and an apartment (9) housing filtration machinery (10, 11), the latter compartment being located above the level of the floatation chamber. The tank can include video equipment and/or means for aerating the floatation solution.

EP 0 128 641 A2

ACTORUM AG

0128641

-1-

The present invention concerns what are known as isolation tanks.the first isolation tank was developed by Dr.John C.Lilley when working for the National Institute for Mental Health(USA) to find out what would happen to the brain if it were deprived of external stimuli.

Isolation tanks function by allowing a user to float in a concentrated solution of Epsom Salts maintained at such a temperature that the user feels neither hot nor cold.This temperature has to be very precise as after prolonged floating variations of over half a degree can cause either sweating or shivering. The correct temperature is aproximately 4 degrees Farenheit below normal body temperature.At the same time the tank user is shielded from all external sounds and the interior of the tank is kept completely dark.The effect of floating in this manner is to counteract as far as is possible the effect of gravity and to releas those energies used to deal with the normally ever-present stimuli of existance and to maintain the three dimensional reference frame which every person has.

The absense of gravity and externally generated stimuli mean that a person using an isolation tank can obtain extremely deep rest and relaxation.the tank user can also channel his or her freed mental energies to positive ends.Isolation tanks can also provide a method of reducing pain in people suffering from injury or illness in a way not readily acheivable by any other non-drug base means.

The present invention has for an object to provide an isolation tank of the general type just described and which is easy to manufacture,compact,and which has greater versatility than previous isolation tanks.

An isolation tank constructed in accordance with the present invention is characterisd in that it is formed from a plurality of moulded thermoplastic elements which are assembled together to provide a compartment for holdong the floatation solution and another compartment in which filtration equipment for the tank is mounted above the level of the floatation solution.

-3-    0128641

In order that the invention may be more readily understood an embodiment theeof will now be described by way of exaple and with reference to the accompanying drawings,in which:

Figure 1 is a perspective view of the rear of an isolation tank according to the invention,

Figure 2 is a view from the front of the same tank with the doors open.

Figure 3 is a digrammatic section through the tank,

Figure 4 is a section showing the flitration equipment of the tank,and

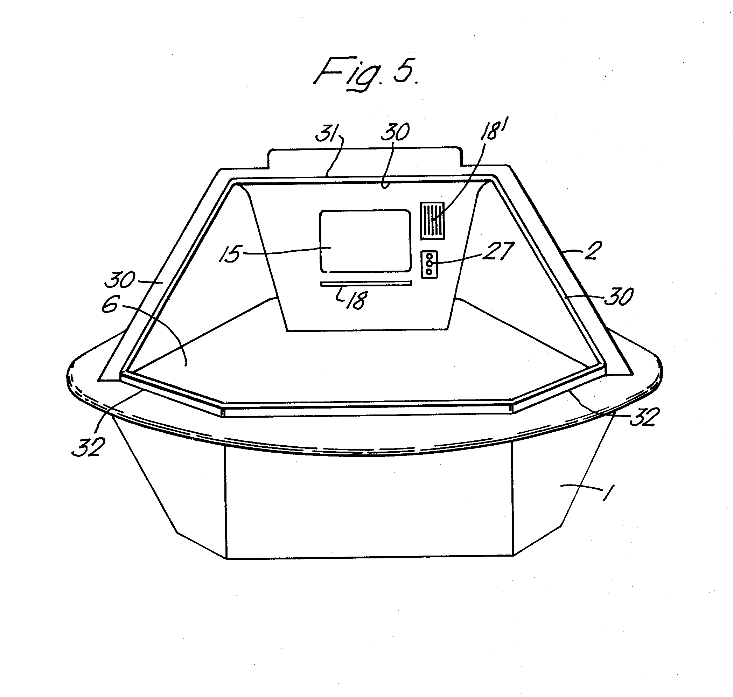

Figure 5 is a view through the open entry door of the tank showing a video screen and a control console.

Referring to the drawings it can be seen that the isolation tank shown is not unlike a boat in shape.The tank is made from five main fibreglass mouldings so designed that in disasembled form each individual element oan easily be passed through an ordinary doorway.

the five elements consist of a lower hull-like element 1 secured to an upper cabin-like element 2.Cabin 2 has a rearwardly sloping portion 3 on which sits a substantially wedge-shaped element or hood 4.Hood 4 is pivoted at 15 so that it can be swung in the direction of arrow A to give access to machinery associated with the operation of the tank.

The hood 4 may thus be provided with one or more handles and with a stay to hold it open.The fourth element is a door 5 provided with a dashpot damping arrangement such that it wil slowly close under its own weight.

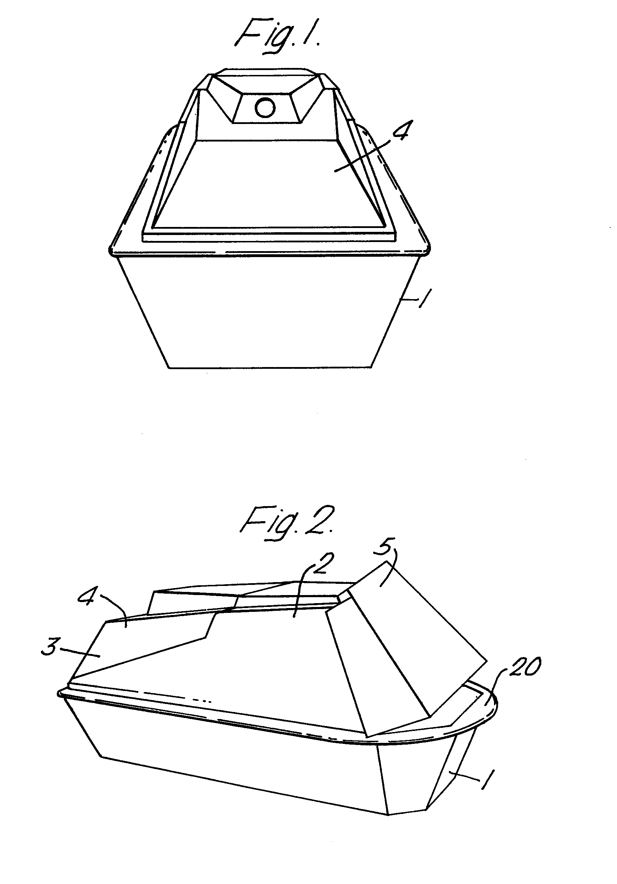

The door 5 and the tank entrance are so arranged that a person getting out of the tank can stand with his or her feet in the floataion solution to allow the solution to drip back into the tank,making it easier to keep the tank surrounds dry.The front part 20 of the tank,as shown in figure 2,provides a ledge on which a user can sit.The entrance is large enough to enable a disabled person to be helped into the tank.

The fifth main lement of the tank is an inner skin 6 which fits within hull 1 and which holds the floatation solution.A heating element 7 passes around the lower periphery of skin 6 in the space between it and hull 1.The position of heating element 7 is of great importance as can be appreciatd from the specification of UK Patent Application no.8014248.The temperature control arrangement can also be as described in this prior specification.

The heating element 7 may be one or more cable-like elements such as are used in heating greenhouses.However the prefered arrangement is to stitch heating elements into fibreglass matting and to mould the structure so formed into the body of inner skin 6 during its manufacture.This simplifies manufacture and makes for a very robust construction.

As seen in figure 2 the interior of the tank is divided into to chambers or compartments,namely a chamber 8 in which the tank user lies,and an equipment compartment 9.Previously all machinery asociated with an isolation has been mounted outside the tank.this has several disadvantages.The floor area taken up is increased,and leaking solution can cause damage.Mounting the machinery within the tank has a numbe of positive advantages.The floataion solution is normally a 26% solution of Epsoms Salts in water.Even with rgular use such a solution can be used without changing for a year provided that it is circulated through the proper filtration equipment.If the latter is situated outside the tank and the ambient tempeature falls the Epsoms Salts may crystallise causing damage to the pumps and filters when they are started again.

In any case the cooled solution,which can amount to several litrea, can cause initial discomfort to a user on being returned to the tank on start up.In the present embodiment the machinery is mounted directly above he floatation solution and is heated by it.The likelyhood of crystallisation is much reduced.

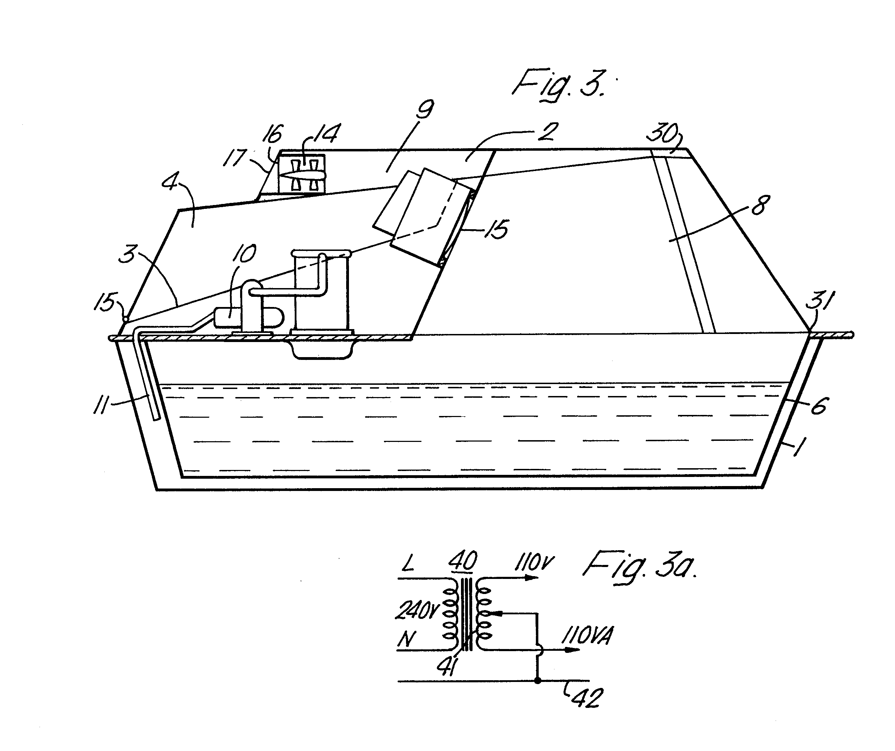

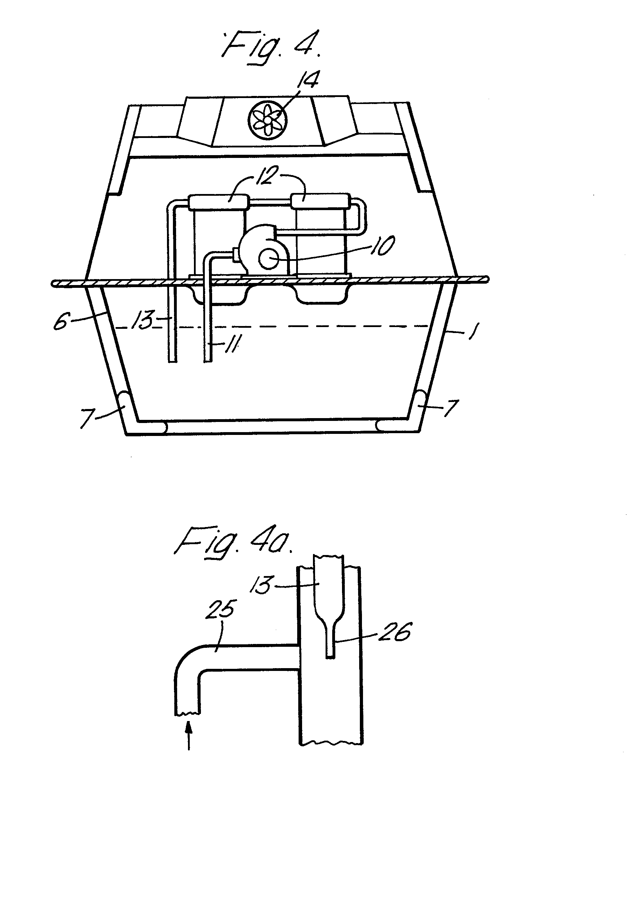

The machinery shown in figures 3 and 4 comprises a pump 10 for drawing solution up through a pipe 11 to successive filtration units 12,the filtered solution being returned to chamber 8 via a return pipe 13.A fan 14 is provided to draw fresh air into the tank and to maintain a slight over-pressure within the tank so that a user gets a constant supply of fresh air without feeling a draught.At its inlet the fan 14 has a particle filter 16 preventing unwanted particles or insects entering the tank and a protective screen 17.Fan 14 is mounted on rubber bearings to stop the noise of the fan reaching a tank user.The inerengaging surfaes of the cabin 2 and the hood 4 are fitted with a rubber sealing strip.

The tank being described has a number of features not found in prior isolation tanks.Thus the tank includes a video unit 15 which can be watched by a person lying in the tank.This is a departure from previous isolation tanks which were intended to shield users from all external stimuli.

The provision of the video unit gives an alternative which is additional to the fundamental purposes of the tank.The environment within the tank is moist so the video unit is mounted behind a screen sealing it from the chamber 8.To stop condensation forming on the screen there is provided a vent 18 which is connected to the over-presure prevailing in the compartment.The vent is adjustable and functions like the demister of a motor car.Another problem caused by the moist environment is that any loudspeaker associated with the video unit will rapidly fail if not protected from the damp.This is done by placing the loudspeaker unit in the path of the warm air supplied to vent 18.To provide communication between a tank user and the outside a microphone can also be placed in the same warm air path.

Another novel feature of the tank being described is that it can be used in a Jacuzzi or spa-type manner.When the tank is used in this way air under pressure is bubbled into the tank.There are several ways in which the solution in the tank can be aerated to give the required bubble effect.One of these is shown in figure 4 in which an air pipe 25 is connected to the return pipe 13.

The filtered solution is pumped by pump 10 to a constriction 26 located at the join between pipes 13 and 25.The escape of the solution from this constriction causes a pressure drop which sucks air into the flowing solution to create the required bubbles.Alternatively a separate source of compresed air such as an air blower can be coupled to supply conduits plumbed into the space between hull 1 and inner skin 6.

The controls for the bubble feature,the video unit and any associated audio unit are mounted in a control panel 27 adjacent the screen of the video unit.

A major problem with all isolation tanks is that when in use moisture condenses on the interior walls and ceiling of the tank to form droplets which in their falling cause irritation to tank users.In the tank being described the slope of the side walls is such that any condensate tends to slip down the side walls without causing any disturbance.The ceiling of the tank is lined with a layer 30 of porous material connected to strips of the same material running down the side walls which act as a gravity feed to draw condensed water from the ceiling back into the main body of the solution.

It is also possible for the whole of the exposed part of the side walls of the floatation chamber to be lined with the same porous material.Thus any area liable to the formation of droplets can be lined with this material.The preferred material is capillary matting.Another feature to combat condensation within the tank is that rebates are formed around the jamb of the door which act both to shield the interior of the tank from external light and as gulleys which drain condensed water to drainage points leading back to chamber 8.These gulleys are shown at 31 and the drainage points at 32.To retain as much heat as possible within the tank and reduce the power consuption of the heating element 7 the side walls of the tank are lined with a composite material of a layer of metallic foil and a layer of closed-cell foam rubber.The latter layer is adhered to the side walls and the metallic layer reflects heat back into the tank.

As the tank includes a substantial amount of electrical equipment safety is of paramount importance.It is possible to use 110 volt heating elements connected via an earth leakage current breaker so as to protect tank users from serious shock.

0128641

However it is preferred to use an isolation transformer of the kind shown in the insert to figure 3.This transformer 40 has its secondary winding 41 centre-tapped and taken to a ground line 42 so as to provide 55 volts on either side of ground for the heating elements.If the remainder of the electrical equipment has an operating voltage of 110 volts then this can be taken fron transformer 40 in the normal manner.With a suitable isolation transformer it means that the tank need not be modified for the US market.

From the foregoing it will be seen that the tank described has a number of positive advantages over prior isolation tanks.Firstly it can provide a number of different facilities.Its power consuption is low because of the compact arrangement of the machinery in an insulated comparment directly above the heated floatation chamber.The fact that the machinery compartment is kept at a slight over-pressure means that if there is a leak or sweating joint then air will tend to flow into the equipment rather than the latter to leak.The double walled tank structure makes for robustness and a further reduction of heat losses as insulation can be inserted between the two walls.The location of the machinery in an isulated compartment makes for very quiet operation.It will finally be appreciated that if the spa function is used it will be necessary to either pre-heat the air used or to draw it from within the tank.

CLAIMS

1. An isolation tank comprising a floatation chamber for holding a sline solution in which a user of the tank can float,and means for maintaining the temperature of the solution at a selected value,the tank being characterised in that it comprises a plurality of moulded plastics elements(1,2,4,5 and 6)assembled to provide a floatation chamber(8) and acompartment(9) housing filtration machinery(10,12),the equipment compartment(9) being located above the level of the floatation solution when the tank is in use.

2. An isolation tank as claimed in claim 1,and further characterised in that it includes video equipment(15) which can be viewed by a user of the tank.

3. An isolation tank as claimed in claim 2,and further characterised in that means(14) are provided to maintain an over-pressure within the equipment compartment(9).

4. An isolation tank as claimed in claim 3,and further characterised in that means(25,26) are provided to aerate the solution within the tank.

5. An isolation tank as claimed in claim 4, and further characterised in that the walls of the equipment compartment are lined with an insulating and reflective material so as to reduce heat losses.

6. An isolation tank as claimed in claim 5, and further characterised in that the walls and ceiling of the floatation chamber(8) are provided with means(30,31,32) for reducing the possibility of droplets of condensed water faling on a user of the tank.

7. An isolation tank as claimed in claim 6, wherein the elements(1,2,4,5,6) from which the main body of the tank is constructed are of such a size that each can be passed through an ordinary doorway.

Fig.1.

Fig.2.

## Fig. 3.

16 14 9 2 30

17

4

3

15

10

15

8

31

11

6

1

## Fig. 3a.

L 40 110v

240v

N

41

110VA

42

*Fig. 4.*

*Fig. 4a.*

*Fig. 5.*